# EUROPÄISCHE PATENTSCHRIFT

(11) **EP 1 970 060 B1**
(45) Veröffentlichungstag und Bekanntmachung des Hinweises auf die Patenterteilung: **29.08.2012**
(21) Anmeldenummer: 08008351.2
(22) Anmeldetag: 26.11.2004
(51) Int. Cl.: A61K 31/35, A61K 31/137, A61K 31/145, A61P 25/22, A61P 25/24, A61P 21/00

(54) **Verwendung von C-(2-Phenyl-cyclohexyl)-methylaminverbindungen zur Therapie von Fibromyalgia**
Use of C-(2-Phenyl-cyclohexyl)-methylamine compounds for fibromyalgia therapy
Utilisation de C-(2-phényle-cyclohexyl)-méthylamines pour le traitement de la fibromyalgie

(30) Priorität: 28.11.2003 DE 10356362
(43) Veröffentlichungstag der Anmeldung: 17.09.2008
(62) Teilanmeldung aus: 04819227.2
(73) Patentinhaber: Grünenthal GmbH, 52078 Aachen (DE)
(72) Erfinder: Bloms-Funke, Petra, Dr., 52146 Würselen (DE); Englberger, Werner, Dr., 52223 Stolberg (DE); Hennies, Hagen-Heinrich, Dr., 52152 Simmerath (DE)
(74) Vertreter: Bülle, Jan

(56) Entgegenhaltungen:
- WO-A-02/43712
- WO-A-02/067651
- WO-A-2004/009067
- WO-A-2004/047823
- WO-A-2006/094961
- WO-A-2007/031326
- DE-A1- 10 254 785
- DE-A1- 19 525 137
- FLICK K ET AL: "UNTERSUCHUNGEN ZUR CHEMISCHEN STRUKTUR UND ANALGETISCHEN WIRKUNG VON PHENYLSUBSTITUIERTEN AMINOMETHYLCYCLOHEXANOLEN STUDIES ON CHEMICAL STRUCTURE AND ANALGETIC ACTIVITY OF PHENYL SUBSTITUTED AMINOMETHYLCYCLOHEXANOLES" ARZNEIMITTEL FORSCHUNG. DRUG RESEARCH, EDITIO CANTOR. AULENDORF, DE, Bd. 28, Nr. 1A, 1978, Seiten 107-113, XP000608150 ISSN: 0004-4172
- LEVENTHAL LAWRENCE: "Management of fibromyalgia" ANNALS OF INTERNAL MEDICINE DEC. 7, 1999 VOL - 131, Bd. 131, Nr. 11, 1999, Seiten 850-858, XP002517722

## Beschreibung

Die Erfindung betrifft die Verwendung von 3-(2-Dimethylaminomethyl-cyclohexyl-phenol zur Herstellung eines Arzneimittels zur Behandlung von Fibromyalgia.

Angststörungen sind Erkrankungen, deren Hauptsymptomatik Manifestationen von unrealistischer bzw. übermäßig ausgeprägter Angst darstellen. Im Falle von Phobien, zu deren Subtypen sogenannte Einfache Phobien, Soziale Angststörungen, Agoraphobien gehören, sind die Angstattacken verbunden mit bestimmten Objekten oder Situationen. Ausgeprägte Angstattacken können aber auch ohne Auslösung durch spezifische Situationen oder Umstände auftreten. So zeichnen sich Panikstörungen durch wiederkehrende, ausgeprägte Angstattacken aus, die nicht vorhersehbar sind und deshalb zur Erwartungsangst führen. Generalisierte Angststörungen sind flottierende, anhaltende Ängste mit vielfältigen, insbesondere vegetativen Symptomen. Patienten, die unter Posttraumatischen Belastungsstörungen (englisch: posttraumatic stress disorders, PTSD) leiden, waren einem kurz- oder langanhaltendem Ereignis oder Geschehen von außergewöhnlicher Bedrohung oder mit katastrophalem Ausmaß ausgesetzt. Dieses Ereignis würde nahezu bei jedem tiefgreifende Verzweiflung auslösen. Betroffene durchleben in einschießenden Bildern die Belastungen immer wieder, einhergehend mit psychovegetativen Symptomen wie u.a. starken Schweißausbrüchen und Herzrasen. Zwangsstörungen (englisch: obsessive compulsive disorders (OCD)) sind gekennzeichnet durch sich wiederholende unangenehme Gedanken, Impulse oder Handlungen, die mehrere Wochen andauern, als zur eigenen Person gehörig erlebt werden und gegen die zumindest partiell Widerstand geleistet wird, da der Betroffene sie als sinnlos empfindet. Sehr häufig bestehen gemischte Angststörungen oder Angststörungen, die mit Depressionen vergesellschaftet sind.

Depressionen sind Störungen der Affektivität, bei denen ein depressives Syndrom im Vordergrund steht, wobei depressiv mit Verstimmung verbunden bzw. traurig gestimmt heißt. Zu den depressiven Erkrankungen werden unipolare schwere Depressionen mit oder ohne Wahn, mittelgradige Depressionen, leichte Depressionen, Dysthymie, Melancholie, bipolare Depressionen (Bipolare Erkrankung I, Manie und schwere Depression; Bipolare Erkrankung II, Hypomanie und schwere Depressionen; zyklothyme Persönlichkeitsstörungen, Hypomanie und milde Depressionen) gezählt.

Zur Therapie von Angststörungen und Depressionen werden solche Medikamente breit eingesetzt, deren anxiolytische und antidepressive Wirkung auf einer Wiederaufnahmehemmung der Monoamine Noradrenalin und / oder Serotonin basieren (Pacher, P., Kohegyi, E., Kecskemeti, V., Furst, S., Current Medicinal Chemistry 2001, 8, 89-100; Goddard, A. W., Coplan, J.D., Gorman, J. M., Charney, D. S., In: Neurobiology of mental illness, Chamey, D.S., Nestler, E.J., Bunney, B.S. (Hrsg.), Oxford Univerity Press, New York, 1999, S. 548-563). Von großem Nachteil ist dabei, dass die Monoaminwiederaufnahmehemmer ihre anxiolytische und antidepressive Wirkung erst nach mehrwöchiger Behandlung entfalten und erst nach ca. 3-4 Wochen ihre volle Wirksamkeit erreichen. Zu Beginn der Behandlung von Angst-, aber auch Depressionspatienten werden durch Standardmedikationen häufig Angstzustände, Unruhe, erhöhte Reizbarkeit sowie Suizidgedanken verstärkt bzw. induziert. Diese psychomotorischen Erregungszustände und Suizidgedanken treten besonders häufig in den ersten Tagen nach Therapiebeginn sowohl von Tricyclischen Antidepressiva, selektiven Serotonin (sog. SSRIs)- als auch gemischten Serontonin-Noradrenalin (sog. SNRIs)- Wiederaufnahmehemmem auf und sind mit einem erhöhten Suizidrisiko verbunden (Jick, H., Kaye, J.A., Jick, S.S.: Antidpressants and the risk of suicidal behaviours, JAMA (2004) 292, 338-343). Daraus ergibt sich die Notwendigkeit zur strengen Überwachung von Patienten, die mit Standardantidepressiva behandelt werden, und ggf. zur Dosisreduktion. Für Angststörungen und Depressionen besteht daher ein hoher Bedarf für eine Therapie, die sich durch einen frühen Wirkeintritt auszeichnet und zu Therapiebeginn keine anxiogenen Nebenwirkungen und damit kein erhöhtes Suizidrisiko auslöst bzw. solche, die durch Antidepressiva induziert sind, hemmt.

Da ca. 20-30 % der Patienten, die an Angststörungen und Depressionen leiden, keine Verbesserung nach Behandlung mit zugelassenen Antidepressiva und Anxiolytika zeigen, sind neue therapeutische Ansätze zur Behandlung bisher pharmakotherapie-resistenter Patienten von hohem Nutzen.

Die zur Therapie von Angststörungen und Depressionen verwendeten Monoaminwiederaufnahmehemmer werden auch für die Behandlung von chronischen Schmerzpatienten verwendet. Neben der eigentlichen antidepressiven und anxiolytischen Wirkungen führen Wiederaufnahmehemmer von Noradrenalin und Serotonin zu einer eigenständigen analgetischen Wirkung, indem absteigende Schmerzhemmbahnen auf der Ebene des Rückenmarks aktiviert werden. Monoaminwiederaufnahmehemmer werden klinisch zur Monotherapie bei Neuropathischen Schmerzen, aber auch als Adjuvanz zu Opiaten für die Behandlung von chronischen Schmerzen (u.a. Entzündungsschmerz, Tumorschmerz, Fibromyalgie) eingesetzt (Sindrup, in: Yaksh, T.L., et al., Anesthesia. Biological foundations. Philadelphia: Lippincott-Raven, 1997, 987-997). Da chronische Schmerzen bei einer Vielzahl von Patienten mit Angststörungen oder Depressionen vergesellschaftet sind, ist eine Substanz mit µ-opiatagonistischen Eigenschaften kombiniert mit einer klinisch relevanten Serotonin- und / oder Noradrenalin-Wiederaufnahmehemmung besonders günstig.
[Y] Möglichkeiten zur Behandlung von Fibromyalgie werden von Leventhal beschrieben (L. J. Leventhal, "Management of fibromyalgia", Ann. Intern. Med., 131 (1999), Seiten 850-858). Verschiedene Opioide zur Anwendung bei der Behandlung von Schmerz sind bereits bekannt aus WO 2004/009067 A1, DE 102 54 785 A1, WO 2007/031326 A2 und WO 2006/094961 A1. Des Weiteren sind 6-Dimethylaminomethyl-1-phenyl-cyclohexanverbindungen zur Anwendung bei der Behandlung von Harninkontinenz geeignet (WO 02/43712 A2).

Aufgabe der vorliegenden Erfindung war es daher, Stoffe, insbesondere Opioidsubstanzen, aufzufinden, die sich zur Therapie von Angststörungen, Depressionen oder gemischten Angst- und Depressionsformen mit oder ohne chronische Schmerzen eignen. Im besonderen sollten Verbindungen mit einem früheren Wirkeintritt im Vergleich zu den bei Angststörungen und Depressionen breit eingesetzten Monoaminwiederaufnahmehemmern gefunden werden.

Überraschenderweise wurde nun gefunden, dass [2-(3-Methoxyphenyl)-cyclohexylmethyl]-dimethylamin wie auch seine Metabolite und dabei insbesondere das 3-(2-Dimethylaminomethyl-cyclohexyl)-phenol über eine therapeutisch relevante anxiolytische und antidepressive Wirkkomponente verfügen, die sich durch einen frühen Wirkeintritt und Fehlen von anxiogenen Effekten auszeichnet. Mechanistische Untersuchungen zeigen den Anteil der µ-opiatagonistischen Komponente an der anxiolytischen ebenso wie antidepressiven Wirkung und insbesondere am frühen Wirkeintritt. Die Substanzen verfügen über ausgeprägte anxiolytische, antidepressive und analgetische Wirkungen und sind somit zur Behandlung von Depressionen, Angststörungen und Schmerzen geeignet. Basierend auf der durch die µ-opiatagonistische Wirkkomponente induzierten Potenzierung der anxiolytischen und antidepressiven Wirkungen, die durch Serotonin- und Noradrenalin-Wiederaufnahmehemmung vermittelt werden, stellt die erfindungsgemäße Verwendung der genannten Verbindungen eine besonders effektive Behandlungsmöglichkeit gerade auch von pharmakoresistenten Angst- und Depressionspatienten dar.

Dementsprechend ist Erfindungsgegenstand die Verwendung von gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate; zur Herstellung eines Arzneimittels zur Behandlung von Fibromyalgia.

Dabei ist es besonders bevorzugt, wenn die verwendete Verbindung als 1 R, 2R Enantiomer vorlieg.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Das bevorzugte Salz der verwendeten Verbindungen ist das Hydrochlorid.

Unter physiologisch verträglich ist zu verstehen, dass die Substanz, insbesondere das Salz als solches, bei Anwendung im Menschen oder Säugetier verträglich ist, also beispielsweise nicht unphysiologisch (z.B. giftig) wirkt.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetierverträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro-1λ⁶-benzo[*d*]isothiazol-3-on (Saccharin), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure. Besonders bevorzugt ist das Hydrochlorid-Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro-1λ⁶-benzo[*d*]isothiazol-3-on (Saccharin), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Kalzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - verträglich ist. Besonders bevorzugt sind die Salze der Alkali- und Erdalkalimetalle aber auch NH₄⁺, insbesondere aber (Mono-) oder (Di-) Natrium-, (Mono-) oder (Di-) Kalium-, Magnesium- oder Kalzium-Salze.

Weiterer Gegenstand der vorliegenden Erfindung ist ein Verfahren zur Behandlung von Angststörungen in einem Säugetier und/oder Menschen, bei dem eine therapeutisch wirksame Menge einer erfindungsgemäß verwendeten Verbindung verabreicht wird.
Dabei ist es von Vorteil, diese Verbindung beim ersten Auftreten von Angststörungen zu verabreichen, da sie einen frühen Wirkeintritt zeigen.

Ebenfalls Gegenstand der vorliegenden Erfindung ist ein Verfahren zur Behandlung von Angststörungen oder Depressionen, bei dem eine erfindungsgemäß verwendete Verbindung als Adjuvanz zu Standardantidepressiva verabreicht wird, um die zu Therapiebeginn von den Antidepressiva ausgelösten psychomotorischen Erregungszustände und das erhöhte Suizidrisiko zu hemmen. Unter Standardantidepressiva im Sinne dieser Erfindung werden alle zugelassenen Antidepressiva verstanden.

Nach den vorliegenden Untersuchungen sind die verwendeten Substanzen und insbesondere (1R,2R)- 3-(2-Dimethylaminomethyl-cyclohexyl)-phenol potente Anxiolytika, Antidepressiva und Analgetika, verfügen also über eine zusätzliche und klinisch relevante anxiolytische Wirkkomponente.

Darüber hinaus können die erfindungsgemäß verwendeten Verbindungen auch zur Herstellung eines Arzneimittels zur Behandlung von Zwangsstörungen, Migräne, Fibromyalgia, Essstörungen, Bulimie, Hyperaktivität, Drogenabhängigkeit, -sucht und -entzug, Trichotillomanie, Tourette's Syndrom, Hauterkrankungen, insbesondere Postherpetische Neuralgie und Pruritus, Psychosen, Gedächtnisstörungen, kognitiven Störungen und/oder Morbus Alzheimer eingesetzt werden.

Geeignete Zusatz- und/oder Hilfsstoffe zu den erfindungsgemäß verwendeten Verbindungen beim Verfahren zur Herstellung des Arzneimittels sind alle dem Fachmann aus dem Stand der Technik bekannten Stoffe zur Erreichung galenischer Formulierungen. Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc. Für Suppositorien können u.a. Wachse bzw. Fettsäureester und für parenterale Applikationsmittel Trägerstoffe, Konservierungsmittel, Suspensionshilfsmittel etc. verwendet werden. Die an Patienten zu verabreichenden Wirkstoffmengen variieren in Abhängigkeit vom Gewicht des Patienten, von der Applikationsart und dem Schweregrad der Erkrankung. Aus oral, rektal oder perkutan anwendbaren Zubereitungsformen können die erfindungsgemäß verwendeten Verbindungen verzögert freigesetzt werden. Bei der erfindungsgemäßen Indikation sind entsprechende Retard-Formulierungen, insbesondere in Form eines "Once-daily"-Präparats, das nur einmal am Tag eingenommen werden muss, besonders bevorzugt.

Bevorzugt sind Arzneimittel, die wenigstens 0,05 bis 90,0 % des Wirkstoffes enthalten, insbesondere niedrige wirksame Dosierungen, um Neben- oder analgetische Wirkungen zu vermeiden. Üblicherweise werden 0,1 bis 5000 mg/kg, insbesondere 1 bis 500 mg/kg, vorzugsweise 2 bis 250 mg/kg Körpergewicht wenigstens einer erfindungsgemäß verwendeten Verbindung appliziert. Ebenso bevorzugt und üblich ist aber auch die Applikation von 0,01 - 5 mg/kg, vorzugsweise 0,03 bis 2 mg/kg, insbesondere 0,05 bis 1 mg/kg Körpergewicht.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat; Pottasche, Calciumphosphat, Dicalciumphosphat, Kaliumbromid, Kaliumiodid, Talkum, Kaolin, Pectin, Crospovidon, Agar und Bentonit.

Die Herstellung der Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur I oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzung zu bilden, die eine erfindungsgemäße Verbindung oder ein pharmazeutisch annehmbares Salz davon in homogener Verteilung enthält. Unter einer homogenen Verteilung wird hier verstanden, dass der Wirkstoff gleichmäßig über die gesamte Zusammensetzung verteilt ist, so dass diese ohne weiteres in gleich wirksame Einheitsdosis-Formen, wie Tabletten, Pillen oder Kapseln, unterteilt werden kann. Die feste Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise compoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Auch wenn die Arzneimittel lediglich geringe Nebenwirkungen zeigen, kann es - so dies überhaupt notwendig sein sollte - beispielsweise zur Vermeidung von bestimmten Formen der Abhängigkeit von Vorteil sein, neben den verwendeten Verbindungen auch Morphinantagonisten, insbesondere Naloxon, Naltrexon und/oder Levallorphan, zu verwenden.

Die Referenzsubstanzen [2-(3-Methoxyphenyl)-cyclohexylmethyl]-dimethylamin und (1R, 2R)-[2-(3-Methoxyphenyl)-cyclohexylmethyl]-dimethylamin und ihre Herstellung sind aus der DE 195 25 137 A1 Beispiel 8 bzw. US 5,733,936 Example 8 bekannt, wobei die absolute Stereochemie der Verbindung (-6) hergestellt nach Beispiel 8 wohl korrekterweise (1R,2R) ist und nicht (1R,2S). Auch 3-(2-Dimethylaminomethyl-cyclohexyl)-phenol bzw. (1 R,2R)-3-(2-Dimethylaminomethyl-cyclohexyl)-phenol und ihre Herstellung sind aus der DE 195 25 137 A1 Beispiel 10 bzw. US 5,733,936 Example 10 bekannt, wobei die absolute Stereochemie der Verbindung (-7) hergestellt nach Beispiel 10 wohl korrekterweise (1R,2R) ist und nicht (1R,2S).

Die Verbindungen, die und deren Herstellung noch nicht aus DE 195 25 137 A1 bzw. US 5,733,936 bekannt sind, wurden gemäß den Beispielen hergestellt.
[Z] Phenylsubstituierte Aminomethylcyclohexanole zur Anwendung bei der Behandlung von Schmerzen werden von Flick et al. ("Untersuchungen zur chemischen Struktur und analgetischen Wirkung von phenylsubstituierten Aminomethylcyclohexanolen", Arzneim.-Forsch.; Drug Res. 28 (1), Heft 1a (1978), Seiten 107-113) beschrieben. Die Anwendung von pharmazeutischen Salzen aus 3-(2-Dimethylaminomethyl-cyclohexyl)-phenol und einem Zuckeraustauschstoff zur Behandlung von Schmerzen werden in WO 02/067651 A1 offenbart.

Die folgenden Beispiele sollen die Erfindung erläutern .

### Beispiele

Generell wurden Reinigung und Enantiomerentrennung bei allen als Beispiel genannten Verfahren auf verschiedenen Stufen mit Säulenchromatographie bzw. überwiegend HPLC, gegebenenfalls an chiralen stationären Phasen, betrieben.

### Beispiel 1: Herstellung von Referenzsubstanz (1R, 2R)-[2-(3-Methoxyphenyl)-cyclohexylmethyl]-methylamin, Hydrochlorid

### [2-(3-Methoxyphenyl)-cyclohexylmethyl]-methylamin, bzw. (1 R, 2R)-[2-(3-Methoxyphenyl)-cyclohexylmethyl]-methylamin, insbesondere deren Hydrochloridsalz, wurden wie folgt hergestellt:

Eine Lösung von 5,67 g (22,9 mmol) (1R,2R)-(2-(3-Methoxyphenyl)-cyclohexylmethyl]-dimethylamin in 390 ml trockenem Toluol wurde in der Siedehitze tropfenweise mit 3,16 ml (25,2 mmol) Chlorameisensäurephenylester versetzt. Nach dreistündigem Erhitzen unter Rückfluss wurde auf 20°C abgekühlt und nacheinander mit je 100 ml Natronlauge (2,5N), destilliertem Wasser, Salzsäure (1N) und einer gesättigten Natriumchloridlösung gewaschen. Es wurde über Natriumsulfat getrocknet und im Vakuum eingedampft. Der Rückstand wurde in 192,5 ml Ethylenglykol und 46 ml Natronlauge (5N) aufgenommen und unter zweimaligem Nachdosieren von je 10 ml Natronlauge (5N) insgesamt 8 Stunden bei 110°C gerührt. Nach Abkühlen wurde mit 100 ml destilliertem Wasser verdünnt und dreimal mit je 50 ml Dichlormethan extrahiert. Die Extrakte wurden mit destilliertem Wasser und einer gesättigten Natriumchloridlösung gewaschen, über Natriumsulfat getrocknet, im Vakuum eingedampft und getrocknet. Der Rückstand (5,03 g) wurde in 32,3 ml 2-Butanon gelöst, die Lösung mit 2,7 ml Trimethylchlorsilan versetzt und 15 Minuten gerührt. Dann wurden 100 ml trockener Diethylether zugegeben, weitere 2 Stunden bei 20°C gerührt und vom Feststoff abgesaugt. Dieser wurde gründlich mit Diethylether gewaschen und im Vakuum getrocknet. Dabei fielen 3,56 g (57,6 % der Theorie) der Titelverbindung in Form farbloser Kristalle an, die bei 165-167°C schmolzen.

### Beispiel 2: Herstellung von Referenzsubstanz (1R,2R)-3-(2-Methylaminomethyl-cyclohexyl)-phenol, Hydrochlorid

3,55 g (15,2 mmol) des Produkts aus Beispiel 1 wurden in 4,59 ml Bromwasserstoffsäure (47-48 % HBr) 7,5 Stunden unter Rückfluss gerührt. Nach Abkühlen wurde mit Eis/Wasser zersetzt, dann mit Natronlauge (6N) alkalisiert. Es wurde dreimal mit je 50 ml Essigsäureethylester extrahiert. Die Extrakte wurden mit einer gesättigten Natriumchloridlösung gewaschen, über Natriumsulfat getrocknet und im Vakuum eingedampft. Der Rückstand (2,91 g) wurde in 16,4 ml 2-Butanon gelöst und mit 1,63 Trimethylchlorsilan wie unter Beispiel 1 beschrieben ins Hydrochlorid überführt. Dabei fielen 2,98 g (76,5 % der Theorie) der Titelverbindung als leicht gelb gefärbter Feststoff an, der bei 173 - 175°C schmolz.

### Beispiel 3: Herstellung von Referenzsubstanz Schwefelsäure mono-(1R,2R)-[3-(2-dimethylaminomethyl-cyclohexyl)-phenyl]ester

Schwefelsäure mono-[3-(2-dimethylaminomethyl-cyclohexyl)-phenyl]ester bzw. Schwefelsäure mono-(1R,2R)-[3-(2-dimethylaminomethyl-cyclohexyl)-phenyl]ester wurden wie folgt hergestellt:

Eine Lösung von 1,00 g (3,92 mmol) (1 R,2R)-3-(2-Dimethylaminomethylcyclohexyl)-phenol; Hydrochlorid und 0,94 g (4,70 mmol) Dicyclohexylcarbodiimid in 20 ml Dimethylformamid wurde bei 0°C unter Rühren tropfenweise mit 15,9 g (4,70 mmol) einer 2,9 %-igen Lösung von Schwefelsäure in Dimethylformamid versetzt. Nach beendeter Zugabe wurde noch weitere 10 Minuten gerührt, dann mit verdünnter Ammoniumhydroxidlösung ein pH-Wert von 9 eingestellt. Der entstandene Niederschlag wurde abgetrennt, mit Essigsäureethylester gewaschen und im Vakuum getrocknet. Man erhielt so 0,26 g (21 % der Theorie) der Titelverbindung als weißen Feststoff.

### Beispiel 4: Herstellung von Referenzsubstanz 6-[(1R,2R)-3-(2-Dimethylaminomethyl-cyclohexyl)-phenoxy]-3,4,5-trihydroxy-tetrahydropyran-2-carbonsäure

6-[3-(2-Dimethylaminomethyl-cyclohexyl)-phenoxy]-3,4,5-trihydroxytetrahydropyran-2-carbonsäure bzw. 6-[(1 R,2R)-3-(2-Dimethylaminomethylcyclohexyl)-phenoxy]-3,4,5-trihydroxy-tetrahydropyran-2-carbonsäure wurden wie folgt hergestellt:

Eine Mischung aus 2,33 g (10 mmol) (1R,2R)-3-(2-Dimethylaminomethylcyclohexyl)-phenol, 3,58 g (9 mmol) Acetobrom-α-D-glucuronsäuremethylester und 0,23 g (9,5 mmol) Lithiumhydroxid in 23 ml trockenem Methanol wurde zunächst 30 Minuten bei 20°C gerührt, dann mit einer Lösung von 0,65 g Lithiumhydroxid in 25 ml Wasser versetzt und nochmals für 30 Minuten gerührt. Man extrahierte mit Essigsäureethylester, stellte durch Zugabe von Essigsäure die wässrige Phase auf einen pH-Wert von 3,5 ein und extrahierte erneut mit Essigsäureethylester. Die Wasserphase wurde im Vakuum eingedampft und der Rückstand durch HPLC gereinigt. Auf diese Weise wurden 0,85 g (23 % der Theorie) der Titelverbindung in Form eines weißen Pulvers erhalten.

### Beispiel 5: Herstellung von Referenzsubstant (1R,2R)-3-(2-Dimethylaminomethylcychlohexyl)-phenol, N-oxid

### 3-(2-Dimethylaminomethyl-cychlohexyl)-pheno, N-oxid bzw. (1 R,2R)-3-(2-Dimethylaminomethyl-cychlohexyl)-phenol, N-oxid wurden wie folgt hergestellt:

Eine Lösung von 5,60 g (24 mmol) (1R,2R)-3-(2-Dimethylaminomethylcyclohexyl)-phenol in 28 ml Methanol wurde mit 10,5 ml Wasserstoffperoxid versetzt und das Gemisch zunächst 3 Stunden bei 50°C, dann 15 Stunden bei 20°C gerührt. Nach Zugabe von 3,30 g Kaliumcarbonat wurde wiederum 3 Stunden gerührt, dann vom Feststoff abfiltriert und das Filtrat im Vakuum eingedampft. Der Rückstand wurde in 30 ml Ethanol aufgenommen. Feste Bestandteile wurden durch Filtration abgetrennt, das Filtrat im Vakuum eingedampft und der Rückstand getrocknet. So wurden 5,50 g (80 % der Theorie) der Titelverbindung in Form eines Öls erhalten, das allmählich erstarrte.

### Beispiel 6: Herstellung von Referenzsubstanz [2-(3-Methoxy-phenyl)-cyclohexylmethyl]-dimethylamin, N-oxid

[2-(3-Methoxy-phenyl)-cyclohexylmethyl]-dimethylamin, N-oxid bzw. (1R,2R)-[2-(3-Methoxy-phenyl)-cyclohexylmethyl]-dimethylamin, N-oxid wurden unter Verwendung entsprechender Einsatzstoffe auf die in Beispiel 5 beschriebene Verfahrensweise hergestellt.

### Beispiel 7: Herstellung von Referenzsubstanz 4-(2-Dimethylaminomethyl-cyclohexyl)-catechol

Die Herstellung von 4-(2-Dimethylaminomethyl-cyclohexyl)-catechol erfolgte nach folgendem Reaktionsschema (Stereochemie nicht berücksichtigt):

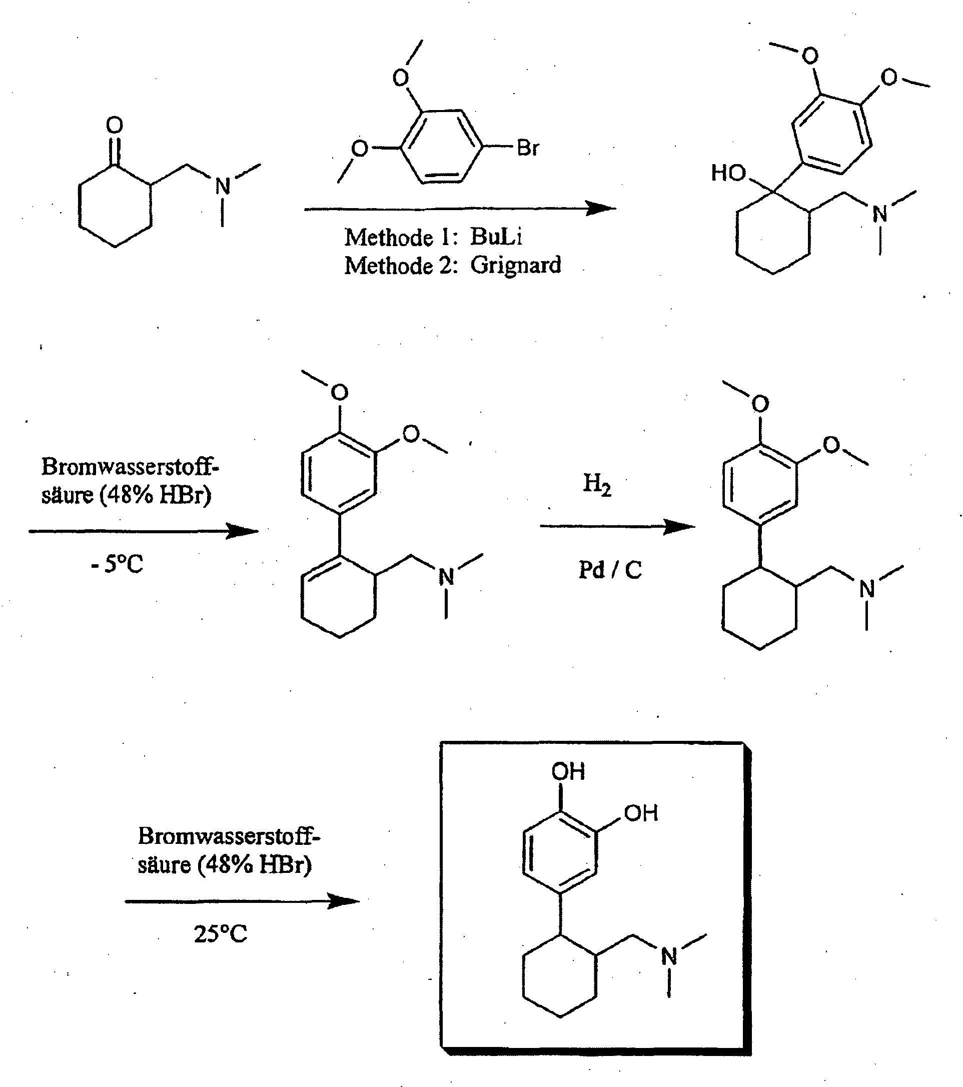

Dabei bezeichnet im übrigen Methode 1 BuLi die dem Fachmann gut bekannte Synthese über den Brom-Lithium-Austausch mit BuLi-Reagenzien und Methode 2 Grignard die dem Fachmann gut bekannte Synthese über Mg-Reagenzien.

### Beispiel 8: Herstellung von Referenzsubstanz C-[2-(3-Methoxy-phenyl)-cyclohexyl]-methylamin.

Die Herstellung von C-[2-(3-Methoxy-phenyl)-cyclohexyl]-methylamin erfolgte nach folgendem Reaktionsschema (Stereochemie nicht berücksichtigt; Bn steht für Benzylrest):

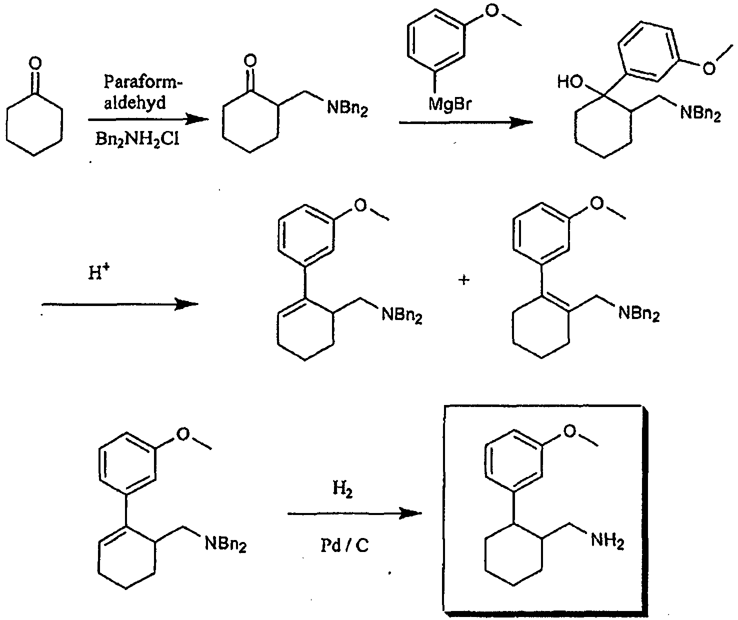

### Beispiel 9: Herstellung von Referenzsubstanz (1R,2R)-3-(2-Aminomethyl-cyclohexyl)-phenol.

Die Herstellung von 3-(2-Aminomethyl-cyclohexyl)-phenol bzw. (1R,2R)-3-(2-Aminomethyl-cyclohexyl)-phenol erfolgte nach folgendem Reaktionsschema:

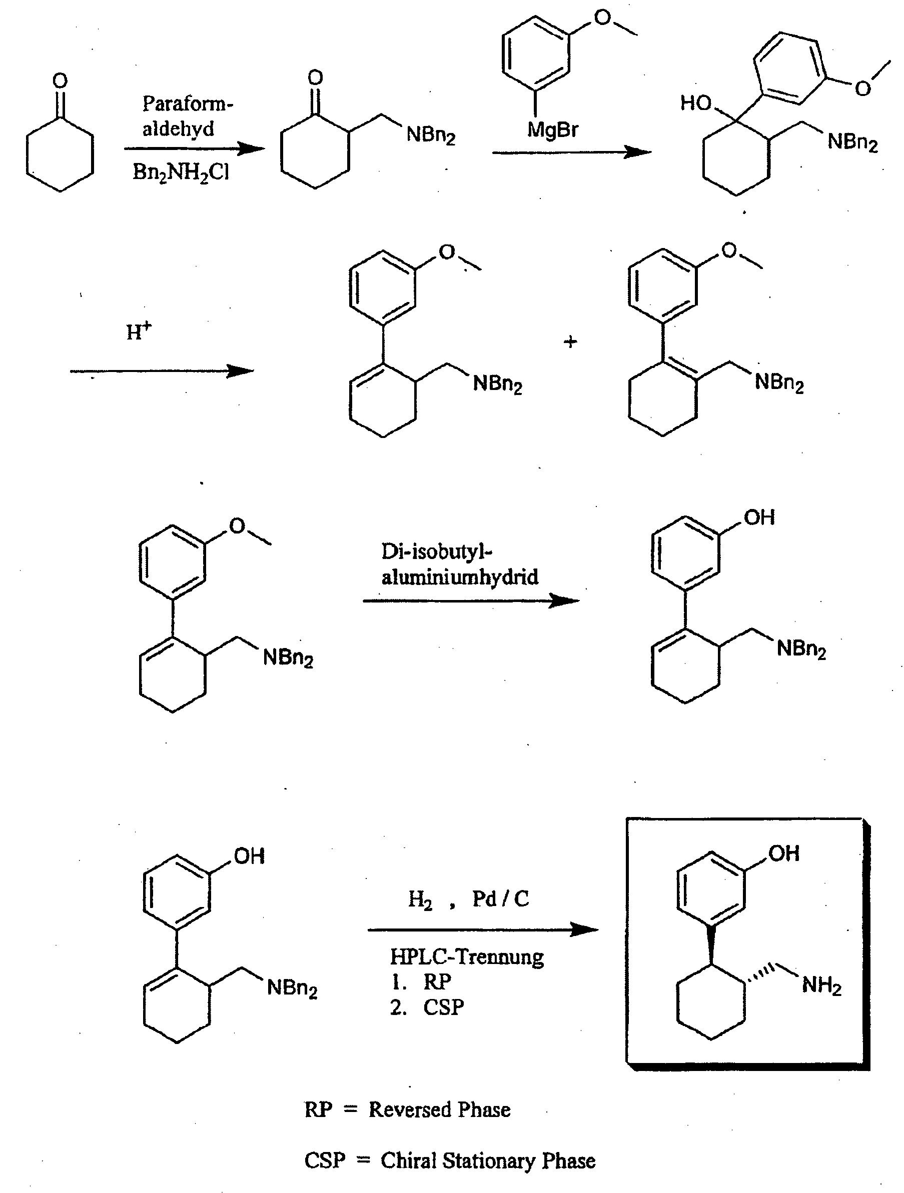

### Beispiel 10 In-Vitro-Isolierung der Metabolite:

Es wurde [2-(3-Methoxyphenyl)-cyclohexylmethyl]-dimethylamin Hydrochlorid Referenz und in einem anderen Beispiel (1R,2R)-3-(2-Dimethylaminomethyl-cychlohexyl)-phenol Hydrochlorid in TRIS/HCl-Puffer pH 7,4 gelöst. Dann wurden MgCl sowie gegebenenfalls die anderen literaturbekannten notwendigen Cofaktoren für CytochromP450 (CytP450) hinzugefügt und mit CytP450 3A4 (N-Demethylierung) und/oder CytP450 2D6 (O-Demethylierung) bei 37°C inkubiert. Anschließend wurde der Ansatz über HPLC aufgetrennt, die Metabolite in den Fraktionen über NMR identifiziert und dann aus den Fraktionen isoliert.

### Beispiel 11 In-Vivo-Isolierung der Metabolite:

Einem Säugetier wurden [2-(3-Methoxyphenyl)-cyclohexylmethyl]-dimethylamin Hydrochlorid Referenz und in einem weiteren Beispiel (1 R,2R)-3-(2-Dimethylaminomethyl-cychlohexyl)-phenol Hydrochlorid injiziert. Dem Säugetier wurde Blut entnommen, dieses nach Abtrennung korpuskulärer Bestandteile über HPLC aufgetrennt, die Metabolite in den Fraktionen über NMR identifiziert und dann aus den Fraktionen isoliert.

### Beispiel 12: Parenterale Applikationsform

1 g (1 R,2R)-3-(2-Dimethylaminomethyl-cyclohexyl)-phenol ; Hydrochlorid wird in 1 I Wasser für Injektionszwecke bei Raumtemperatur gelöst und anschließend durch Zugabe von NaCl auf isotone Bedingungen eingestellt.

### Pharmakologische Untersuchungen

### a) Methoden zur Bestimmung der Affinität zum humanen µ-Opiatrezeptor und der 5-HT- und NA-Wiederaufnahmehemmung

### Untersuchung der Affinität zum humanen µ-Opiatrezeptor

Die Rezeptoraffinität zum humanen µ-Opiatrezeptor wird in einem homogenen Ansatz in Mikrotiterplatten bestimmt. Hierzu werden Verdünnungsreihen der zu prüfenden Substanzen mit einer Rezeptormembranpräparation (15 - 40 µg Protein / 250 µl Inkubationsansatz) von CHO-K1-Zellen, welche den humanen µ-Opiatrezeptor exprimieren (RB-HOM-Rezeptormembran-Präparation von Fa. Perkin Elmer, Zaventem, Belgien) in Gegenwart von 1 nmol/l des radioaktiven Liganden [³H]-Naloxon (NET719, Fa. Perkin Eimer, Zaventem, Belgien) sowie von 1 mg WGA-SPA-Beads (Wheat germ agglutinin SPA Beads der FA. Amersham/Pharmacia, Freiburg, Deutschland) in einem Gesamtvolumen von 250 µl für 90 Minuten bei Raumtemperatur inkubiert. Als Inkubationspuffer wird 50 mmol/l Tris-HCl supplementiert mit 0,05 % Natriumazid und mit 0,06 % bovinem Serumalbumin verwendet. Zur Bestimmung der unspezifischen Bindung wird zusätzlich 25 µmol/l Naloxon zugegeben. Nach Beendigung der neunzigminütigen Inkubationszeit werden die Mikrotiterplatten für 20 Minuten bei 1000 g abzentrifugiert und die Radioaktivität in einem β-Counter (Microbeta-Trilux, Fa. PerkinElmer Wallac, Freiburg, Deutschland) vermessen. Es wird die prozentuale Verdrängung des radioaktiven Liganden aus seiner Bindung zum humanen µ-Opiatrezeptor bei einer Konzentration der Prüfsubstanzen von 1 µmol/l bestimmt und als Prozent Hemmung der spezifischen Bindung angegeben. Ausgehend von der prozentualen Verdrängung durch unterschiedliche Konzentrationen der Prüfsubstanzen werden IC₅₀ Hemmkonzentrationen berechnet, die eine 50-prozentige Verdrängung des radioaktiven Liganden bewirken. Durch Umrechnung mittels der Cheng-Prusoff-Beziehung werden Kᵢ-Werte für die Prüfsubstanzen erhalten (Cheng und Prusoff 1973).

### Untersuchungen der 5-HT- und NA-Wiederaufnahmehemmung

Um diese in vitro Studien durchführen zu können, werden Synaptosomen aus Rattenhimarealen frisch isoliert. Es findet jeweils eine so genannte "P₂"-Fraktion Verwendung, die exakt nach der Vorschrift von Gray und Whittaker (1962) präpariert wird. Für die NA- Wiederaufnahme werden diese vesikulären Partikel aus dem Hypothalamus, für die 5-HT-Wiederaufnahme aus der Medulla + Pons-Region von männlichen Rattengehirnen isoliert.

Folgende Kenndaten wurden für die NA- und 5-HT- Wiederaufnahme ermittelt:
NA-Uptake: Km = 0,32 ± 0,11 µM
5-HT-Uptake: Km = 0,084 ± 0,011 µM
(Jeweils N = 4, d.h. Mittelwerte ± SEM aus 4 unabhängigen Versuchsreihen, die in Dreifach-Parallelversuchen durchgeführt wurden).

Eine detaillierte Methodenbeschreibung ist in der Publikation von Frink, Hennies, Englberger et al. (1996) enthalten (der Ansatz kann auch auf Mikrotiterplatten (250 µl / well) bei Zimmertemperatur durchgeführt werden).

### Auswertungen:

Neben % Hemmungen bei fixen Testsubstanzkonzentrationen (z.B. 1 x 10⁻⁶ M oder 1 x 10⁻⁵ M im Ansatz), wurden Dosisabhängigkeiten überprüft. Hierbei werden IC₅₀-Werte erhalten, die gemäß der "Cheng-Prusoff Gleichung" (Cheng und Prusoff 1973) in Inhibitorkonstanten (Kᵢ) umgerechnet werden können. Die IC₅₀ Werte wurden mit Hilfe des Computer-Programms "Figure P" (Version 6.0, Biosoft, Cambridge, England) erhalten. Km-Werte wurden gemäß Lineweaver und Burk (1934) berechnet. Um K_{D}-Werte darzustellen, ist das Computer-Programm "Ligand" (Version 4, Biosoft, England) angewendet worden.

### Literatur:

- Frink; M. Ch., Hennies, H.-H., Englberger, W., Haurand, M. und Wilffert, B. (1996) Arzneim.-Forsch./Drug Res. 46 (II), 11, 1029-1036
- Gray, E.G. und Whittaker,V.P. (1962)J. Anat. 76, 79-88
- Cheng, Y.C. und Prusoff, W. H. (1973) Blochem. Pharmacol. 22, 3099-3108
- Lineweaver, H. und Burk, D. (1934) J. Am. Chem. Soc. 56, 658-666

Für die erfindungsgemäßen Verbindungen wurde ein deutliche Affinität zum µ-Opiatrezeptor und eine Hemmung der Serotonin- oder Noradrenalinwiederaufnahme gemessen. Insbesondere wurde ein ausbalanciertes Verhältnis zwischen der µ-Opioidkomponente und der Monoaminwiederaufnahmehemmung gefunden, wobei letztere in der Größenordnung von klinisch verwendeten Substanzen lag. Daher besitzt die Verbindung 3-(2-Dimethylaminomethyl-cyclohexyl)-phenol ein sehr vielversprechendes Potential für die Verwendung als Anxiolytikum, Antidepressivum und Analgetikum.

Die Ergebnisse von Beispielen sowie für die Referenzsubstanzen Venlafaxin sind in der nachfolgenden Tabelle dargestellt.

**Tabelle 1**

| **Verbindung** | **µ-Opioidrezeptor-Affinität (Ki-Werte, µmol/l)** | **5-HT- Wiederaufnah-mehemmung (Ki-Werte, µmol/l)** | **NA-Wiederaufnahmehemmung (Ki-Werte, µmol/l)** |
|---|---|---|---|
| 3-(2-Dimethylaminomethylcyclohexyl)-phenol, Hydrochlorid | 0,14 | 0,05 | 0,16 |
| Vergleichsbeispiel 3-(2-Methylaminomethyl-cyclohexyl)-phenol, Hydrochlorid | 0,87 | 5,67 | 0,48 |
| Vergleichsbeispiel Venlafaxin | unwirksam | 0,062 | 0,45 |

### b) Untersuchung des Wirkeintritts anxiolytischer Wirkungen im Elevated plus maze-Test an der Ratte

Im Elevated plus maze-Test werden Substanzwirkungen auf die endogene Angst von Nagern vor offenen und erhöhten Plätzen bestimmt.

Die Apparatur wurde ca. 1 m über dem Boden aufgestellt und bestand aus vier Armen, die im Kreuz angeordnet waren, wobei zwei gegenüberliegende Arme offen und zwei geschlossen waren. Die Ratten wurden einzeln in das zentrale quadratische Kompartiment gesetzt, von dem aus der Zugang zu allen vier Armen möglich war, und das Verhalten der Tiere wurde für 5 Minuten beobachtet. Ausgewertet wurde die Aufenthaltsdauer sowie die Zahl der Entritte in die offenen Arme. Die Gruppengröße betrug 10-15 Tiere. Die Einmalgabe der Prüfsubstanzen bzw. des Kontrollvehikels erfolgte 30 min vor der Testung.

In der Literatur ist beschrieben, dass Benzodiazepine eine erhöhte Aufenthaltsdauer und Eintritte in das offene Kompartiment induzieren. Im Gegensatz dazu bewirken Antidepressiva, deren Hauptmechanismus die Wiederaufnahmehemmung der Monamine Serotonin und / oder Noradrenalin ist, nach Einmalgabe in dem Elevated plus maze-Test keine anxiolytische Wirkung, sondern in einigen Fällen anxiogene Effekte, und erst nach chronischer Gabe über 2-4 Wochen können anxiolytische Effekte beobachtet werden (Borsini, F., Podhoma, J., Marazziti, D., Psychopharmacology, 2002, 163, S. 121-141). Somit lassen sich in dem Elevated plus maze-Test an der Ratte die in der Therapie von Angstpatienten typischen Nachteile der Monoaminwiederaufnahmehemmer, nämlich der verzögerte Wirkeintritt und die initial anxiogenartigen Effekte, nachstellen. Daher stellt der Elevated plus maze-Test ein geeignetes Tiermodell für die Untersuchung neuer Therapien dar, die eine Beschleunigung des Wirkeintritts von Monoaminwiederaufnahmehemmern zum Ziel haben.

Nach Einmalgabe der Verbindung 3-(2-Dimethylaminomethyl-cyclohexyl)-phenol wurden eine signifikante Erhöhung der Aufenthaltsdauer und eine deutliche Erhöhung der Anzahl der Eintritte in die offenen Arme und somit signifikante anxiolytische Wirkungen gemessen. In keinem Fall wurde eine anxiogen-artige Wirkung nach akuter Gabe des Beispiels 3-(2-Dimethylaminomethyl-cyclohexyl)-phenol ausgelöst. Wurde 3-(2-Dimethylaminomethyl-cyclohexyl)-phenol mit dem µ-Opiatrezeptorantagonisten Naloxon kombiniert, so ließen sich dadurch die anxiolytischen Effekte von 3-(2-Dimethylaminomethyl-cyclohexyl)-phenol komplett aufheben. Die Naloxon-Sensitivität der anxiolytischen Effekte von 3-(2-Dimethylaminomethyl-cyclohexyl)-phenol belegen, dass die Opiatkomponente von 3-(2-Dimethylaminomethyl-cyclohexyl)-phenol für den frühen Wirkeintritt nach Einmalgabe der Substanz maßgeblich ist.

Diazepam bewirkte eine Erhöhung der Aufenthaltsdauer und der Eintritte in die offenen Arme. Der gemischte Serotonin- und Noradrenalin-Wiederaufnahmehemmer Venlafaxin zeigte keine anxiolytische Wirkung nach Einmalgabe.

Die Ergebnisse für 3-(2-Dimethylaminomethyl-cyclohexyl)-phenol sowie für die Referenzsubstanzen Diazepam und Venlafaxin sind in der nachfolgenden Tabelle dargestellt.

**Tabelle 2**

| | | Offene Arme | |
|---|---|---|---|
| Substanz | Dosis (mg/kg i.p.) | Aufenthaltsdauer (sec) | Zahl der Eintritte |
| Vehikel | - | 16,8 | 2,2 |
| 3-(2-Dimethylaminomethyl-cyclohexyl)-phenol | 8 | 29,0 | 3,2 |
| | 16 | 118,2 * | 8,5 |
| 3-(2-Dimethylaminomethyl-cyclohexyl)-phenol + Naloxon | 16 + 1 | 37,1 # | 1,6 |
| Naloxon | 1 | 28,6 | 4,5 |
| Vergleichsbeispeil Diazepam | 2 | 77,7* | 8,1* |
| | | | |
| Vehikel | - | 41,6 | 4,3 |
| Vergleichsbeispiel Venlafaxin | 16 | 66,9 | 4,6 |

Statistische Auswertung: Anova plus post-hoc Dunnett's Test (Signifikanzniveau: p< 0,05; *: signifikant versus Vehikel; #: signifikant versus Eigeneffekt von 16 mg/kg i.p. 3-(2-Dimethylaminomethyl-cyclohexyl)-phenol

Bevorzugte Ausführungsformen 1 bis 5 der Erfindung sind nachfolgend zusammengefasst:

Ausführungsform 1: Verwendung von C-(2-Phenyl-cyclohexyl)-methylaminverbindungen, ausgewählt aus
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
zur Herstellung eines Arzneimittels zur Behandlung von Fibromyelgia.

Verwendung gemäß Ausführungsform 1, dadurch gekennzeichnet, dass die verwendeten Verbindung als 1 R, 2R Enantiomere vorliegt.

## Patentansprüche

1. 3-(2-Dimethylaminomethyl-cyclohexyl)-phenol,
ggf. in Form des Racemats, der reinen Stereoisomere, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form der Säure oder Base oder in Form der physiologisch verträglichen Salze, oder in Form der Solvate, insbesondere der Hydrate;
zur Anwendung bei der Behandlung von Fibromyalgia.

2. 3-(2-Dimethylaminomethyl-cyclohexyl)-phenol zur Anwendung nach Anspruch 1, **dadurch gekennzeichnet, dass** die Verbindung als 1R,2R Enantiomer vorliegt.

3. 3-(2-Dimethylaminomethyl-cyclohexyl)-phenol zur Anwendung nach Anspruch 1 oder 2, **dadurch gekennzeichnet, dass** die Behandlung von Schmerzen erfolgt.

4. 3-(2-Dimethylaminomethyl-cyclohexyl)-phenol zur Anwendung nach einem der vorstehenden Ansprüche, **dadurch gekennzeichnet, dass** die Behandlung von Angststörungen oder Depressionen erfolgt.

## Claims

1. 3-(2-Dimethylaminomethyl-cyclohexyl)-phenol,
optionally in the form of the racemate, the pure stereoisomers, in particular enantiomers or diastereomers, or in the form of mixtures of the stereoisomers, in particular the enantiomers or diastereomers, in any desired mixture ratio;
in the form shown or in the form of the acid or base or in the form of the physiologically acceptable salts, or in the form of the solvates, in particular the hydrates;
for use in the treatment of fibromyalgia.

2. 3-(2-Dimethylaminomethyl-cyclohexyl)-phenol for the use according to claim 1, **characterized in that** the compound is present as the 1R,2R enantiomer.

3. 3-(2-Dimethylaminomethyl-cyclohexyl)-phenol for the use according to claim 1 or 2, **characterized in that** treatment of pain takes place.

4. 3-(2-Dimethylaminomethyl-cyclohexyl)-phenol for the use according to one of the preceding claims, **characterized in that** treatment of anxiety disorders or depressions takes place.

## Revendications

1. 3-(2-diméthylaminométhylcyclohexyl)-phénol, le cas échéant sous forme du racémate, des stéréo-isomères purs, en particulier des énantiomères ou diastéréo-isomères, ou sous forme de mélanges des stéréo-isomères, en particulier des énantiomères ou des diastéréo-isomères, dans un rapport de mélange quelconque ;
sous la forme préparée ou sous forme des acides ou des bases ou sous forme des sels physiologiquement acceptables, ou sous forme des solvates, en particulier des hydrates ;
pour l'utilisation dans le traitement de la fibromyalgie.

2. 3-(2-diméthylaminométhylcyclohexyl)-phénol pour l'utilisation selon la revendication 1, **caractérisé en ce que** le composé se trouve sous forme d'énantiomère 1R,2R.

3. 3-(2-diméthylaminométhylcyclohexyl)-phénol pour l'utilisation selon la revendication 1 ou 2, **caractérisé en ce que** l'on réalise le traitement de la douleur.

4. 3-(2-diméthylaminométhylcyclohexyl)-phénol pour l'utilisation selon l'une quelconque des revendications précédentes, **caractérisé en ce que** l'on réalise le traitement de troubles d'anxiété ou de dépressions.
